# EUROPEAN PATENT APPLICATION

(11) **EP 4 509 121 A1**
(43) Date of publication of application: **19.02.2025**
(21) Application number: 23788385.5
(22) Date of filing: 13.04.2023
(51) Int. Cl.: A61K 31/198, A23L 19/00, A23L 33/175, A61P 43/00

(54) **ENAMPT INCREASING AGENT, SIRTUIN ACTIVATION OR EXPRESSION ENHANCER, NAD+ INCREASING AGENT, AND SENESCENT CELL INHIBITOR**

(30) Priority: 13.04.2022 WO PCT/JP2022/017713; 12.10.2022 JP 2022164310
(71) Applicant: Wakunaga Pharmaceutical Co., Ltd., Osaka-shi, Osaka 532-0003 (JP)
(72) Inventor: SUZUKI Junichiro, Akitakata-shi, Hiroshima 739-1195 (JP); KUNIMURA Kayo, Akitakata-shi, Hiroshima 739-1195 (JP); TAKASHIMA Miyuki, Akitakata-shi, Hiroshima 739-1195 (JP); MIKI Satomi, Akitakata-shi, Hiroshima 739-1195 (JP); USHIJIMA Mitsuyasu, Akitakata-shi, Hiroshima 739-1195 (JP); OHNO Shohei, Akitakata-shi, Hiroshima 739-1195 (JP)
(74) Representative: Ström & Gulliksson AB
(86) International application number: PCT/JP2023/014975
(87) International publication number: WO 2023/199967

(57) **Abstract**

Provided is a novel use of a composition that contains S-1-propenylcysteine and/or a salt thereof.

The present invention can be an eNAMPT increasing agent in which S-1-propenylcysteine or a salt thereof is an active ingredient. The present invention can be an eNAMPT increasing agent that contains S-1-propenylcysteine or a salt thereof and that is administered to an animal. Particularly, it is preferable that the present invention be used for increasing eNAMPT in blood.

## Description

### TECHNICAL FIELD

The present invention relates to an agent used for increasing eNAMPT, activating or enhancing expression of sirtuins, increasing NAD⁺, and suppressing senescent cells.

### BACKGROUND ART

Sirtuins are known as molecules, for example, in nematodes, flies, and mammals, whose high expression can prolong lifespan while deficiency thereof can shorten such lifespan. Genes of sirtuins that are NAD⁺ dependent deacetylases are widely conserved from bacteria to eukaryotes. Sirtuins have been attracting attention as candidates for longevity genes in animals, because deficiency of Sir2, which is a sirtuin homolog of yeast and nematodes, shortens the lifespan, while overexpression of Sir2 prolongs the lifespan. There are seven sirtuins (SIRT1 to SIRT7) in mammals, and among them, SIRT1 having a structure and function that is the most similar to yeast Sir2 has been revealed to be probably involved in controlling a wide range of cellular functions such as gene expression associated with aging, intracellular metabolism, energy consumption, inflammation, and stress response pathways (Non-Patent Literature 1). In recent years, it has been revealed that sirtuins may be related to specific diseases. For example, Non-Patent Literature 2 shows that cognitive functions including immediate memory, classical conditioning, and spatial learning are impaired in SIRT1 knockout mice. Conversely, overexpression of SIRT1 is observed to exhibit ordered synaptic plasticity and memory. It has been revealed that SIRT1 acts on normal learning, memory, and synaptic plasticity. Furthermore, Non-Patent Documents 3 and 4 describe relationships of SIRT1 with liver steatosis and cardiac function respectively.

Nicotinamide mononucleotide (NAD⁺) functions as a coenzyme for dehydrogenases and a substrate for sirtuins in living bodies. It is also known that an increase in NAD⁺ amount enhances sirtuins activity. Synthesis of NAD is controlled by nicotinamide phosphoribosyltransferase (NAMPT). It is known that decreased function of NAMPT with age leads to reduced NAD⁺ amount and decreased sirtuins activity. Non-Patent Document 5 discloses that administration of nicotinamide riboside, which is a precursor of NAD⁺, suppresses cellular senescence in brain. Non-Patent Documents 6 and 7 describe relationships of NAD⁺ with nonalcoholic fatty liver diseases and kidney injuries respectively.

Cellular senescence is a phenomenon of the occurrence of irreversible cell cycle arrest that results from excessive DNA damage via accumulation of DNA replication errors associated with cell division, oxidative stress, radiation, activation of oncogenes, or the like while activating p16/RB pathways and p53/p21 pathways, and inducing inhibitors of cyclin inhibitor kinase. Cells that have undergone the cellular senescence (the senescent cells) accumulate in tissues due to accelerated cellular senescence and reduced removal ability caused by decline in mitochondria and immune functions associated with aging. The senescent cells accumulated in tissues secrete, for example, inflammatory cytokines and proteases, which are called cellular senescence associated secretory factors, thereby damaging surrounding tissues and accelerating aging of tissues and living bodies. On the other hand, it has been suggested that removal of senescent cells may prevent or delay tissue dysfunction to suppress the senescence (Non-Patent Document 8). Furthermore, it has been revealed that an increase in senescent cells is involved in kidney injuries (Non-Patent Document 9) and fatty liver (Non-Patent Document 10).

On the other hand, S-1-propenylcysteine is a cysteine derivative represented by the following Formula (1), and is one of sulfur-containing components that can be found in plants of genus Allium such as garlic.

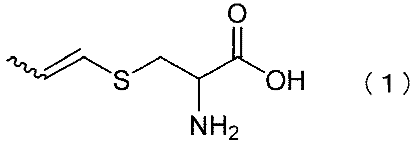

It has been reported that S-1-propenylcysteine has pharmacological actions such as immune function regulating action (Patent Literature 1), antihypertensive action (Patent Literature 2), antioxidant action (Patent Literature 3), blood flow improving action (Patent Literature 4), autophagy activation action (Patent Literature 5), and preventing, treating, or ameliorating actions on periodontal disease (Patent Literature 6). However, nothing is known about increase in eNAMPT, activation of sirtuins, suppression of senescent cells, and increase in NAD⁺ by S-1-propenylcysteine.

### Citation List

### Patent Document

Patent Document 1: PCT International Publication No. WO2016/088892
Patent Document 2: PCT International Publication No. WO2016/199885
Patent Document 3: Japanese Unexamined Patent Application, Publication No. 2020-029529
Patent Document 4: PCT International Publication No. WO2019/031442
Patent Document 5: PCT International Publication No. WO2019/235597
Patent Document 6: PCT International Publication No. WO2020/230813

### Non-Patent Document

Non-Patent Document 1: Trends Cell Biol. 2014;24(8):464-71.
Non-Patent Document 2: J Neurosci 2010;30(29):9695-9707.
Non-Patent Document 3: Molecular Medicine Reports 2018;18:1609-1615
Non-Patent Document 4: Aging 2021;13(10):14482-14498
Non-Patent Document 5: Cell Metab. 2018 Mar 6;27(3):513-528.
Non-Patent Document 6: British Journal of Pharmacology 2016;173:2352-2368
Non-Patent Document 7: Nature 2016;531(7595):528-532
Non-Patent Document 8: Nature. 2011 Nov 2;479(7372):232-6.
Non-Patent Document 9: Front Pharmacol. 2019;10:770.
Non-Patent Document 10: Nature Communications 2017;8:15691

### DISCLOSURE OF THE INVENTION

### Problems to be Solved by the Invention

The present invention relates to providing a new use of S-1-propenylcysteine, a salt thereof, or a composition containing the same.

### Means for Solving the Problems

The present invention relates to the following 1) to 11).
1) An eNAMPT increasing agent containing S-1-propenylcysteine or a salt thereof as an active ingredient.
2) An eNAMPT increasing agent containing S-1-propenylcysteine or a salt thereof that is administered to an animal.
3) The agent according to 2) that is used for increasing eNAMPT in blood.
4) An agent containing S-1-propenylcysteine or a salt thereof that is administered to an animal and is used for activating or enhancing expression of sirtuins, or suppressing senescent cells in one or more organs.
5) An agent containing S-1-propenylcysteine or a salt thereof that is administered to an animal and is used for increasing NAD⁺ in one or more organs.
6) The agent according to 4) or 5), in which the organ includes at least one selected from a group consisting of brain, kidney, skeletal muscle, liver, and heart.
7) The agent according to 6), in which the organ includes at least one selected from the group consisting of skeletal muscle, liver, and heart.
8) The agent according to 7), in which the organ further includes at least one selected from the group consisting of brain and kidney.
9) The agent according to any one of 4) to 8) for acting on two or more organs.
10) The agent according to 9) for acting on three or more organs.
11) The agent according to any one of 1) to 10) being in a form of a food additive or food.

### Effects of the Invention

The present invention provides a new use of S-1-propenylcysteine, a salt thereof, or a composition containing the same.

### BRIEF DESCRIPTION OF THE DRAWINGS

FIG. 1 illustrates a sirtuin activation action in a cerebral cortex by administrating S-1-propenylcysteine;
FIG. 2 illustrates a change in a SIRT1 protein mass in a hippocampus by administrating S-1-propenylcysteine;
FIG. 3 illustrates changes in NAD⁺ amount in a hippocampus by administrating S-1-propenylcysteine;
FIG. 4 illustrates a change in p53 protein that is a senescent cell marker in a hippocampus by administrating S-1-propenylcysteine;
FIG. 5 illustrates a change in expression level of SIRT1 in a kidney by administrating S-1-propenylcysteine;
FIG. 6 illustrates changes in expression levels of p16 and p21 genes that are senescent cell markers in a kidney by administrating S-1-propenylcysteine;
FIG. 7 illustrates changes in KIM-1 and NGAL gene protein masses that are markers of kidney injuries in a kidney by administrating S-1-propenylcysteine;
FIG. 8 illustrates cognition/memory retention by administrating S-1-propenylcysteine;
FIG. 9 illustrates changes in NAD⁺ amounts in a kidney, a skeletal muscle, and a liver by administrating S-1-propenylcysteine;
FIG. 10 illustrates changes in SIRT1 protein masses in a cerebral cortex, a hippocampus, a heart, a lung, a liver, a kidney, and a skeletal muscle by administrating S-1-propenylcysteine;
FIG. 11 illustrates a change in a SIRT1 protein mass in a hypothalamus by administrating S-1-propenylcysteine;
FIG. 12 illustrates a change in a blood eNAMPT amount by administrating S-1-propenylcysteine; and
FIG. 13 illustrates changes in SIRT1 protein masses in a liver and a heart by administrating S-1-propenylcysteine.

### PREFERRED MODE FOR CARRYING OUT THE INVENTION

### <Agent>

"S-1-propenylcysteine" has a cis or a trans configuration as indicated by a wavy line in Formula (1) below. In the present invention, it is preferable that S-1-propenylcysteine has a large ratio of trans isomer, and when the total of the trans isomer and cis isomer is taken as 100%, a ratio of the trans isomer is more preferably 50 to 100%, more preferably 75 to 100%, more preferably 80 to 100%, and still more preferably 90 to 100%. In addition, existence of an asymmetric carbon in a cysteine structure leads to existence of an optical isomer. However, it may be any of a D isomer, an L isomer, or a racemic form.

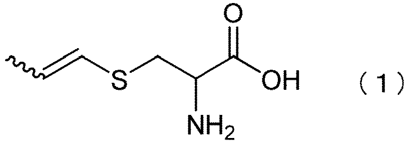

A salt of S-1-propenylcysteine is a physiologically acceptable salt, and may be either an acid addition salt or a base addition salt. Examples of the acid addition salt include (a) salts with mineral acids such as hydrochloric acid, sulfuric acid, nitric acid, or phosphoric acid, (b) salts with organic carboxylic acids such as formic acid, acetic acid, citric acid, fumaric acid, gluconic acid, malic acid, succinic acid, tartaric acid, trichloroacetic acid, or trifluoroacetic acid, and (c) salts with sulfonic acids such as methanesulfonic acid, benzenesulfonic acid, p-toluenesulfonic acid, mesitylenesulfonic acid, or naphthalenesulfonic acid; and examples of the base addition salt include (a) salts with alkali metals such as sodium or potassium, (b) salts with alkaline earth metals such as calcium or magnesium, (c) ammonium salts, and (d) salts with nitrogen-containing organic bases such as trimethylamine, triethylamine, tributylamine, pyridine, N, N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine.

Furthermore, S-1-propenylcysteine or a salt thereof can exist not only in an unsolvated form but also as a hydrate or a solvate, and such hydrate or solvate can be any crystal form depending on production conditions. Therefore, S-1-propenylcysteine or a salt thereof in the present invention includes all stereoisomers, hydrates, solvates, and all polymorphic crystalline or amorphous forms.

S-1-propenylcysteine or a salt thereof can be obtained by an organic synthesis method ([1] Tetrahedron Letter, 1975, 37, 3201-3202; [2] Synthesis, 2006, 20, 3367-3369; [3] Bull. Korean Chem. Soc. 2011, 32(1), 319-320). In addition, S-1-propenylcysteine or a salt thereof can be obtained by processing a plant of genus Allium by various methods. Examples include a method of obtaining S-1-propenylcysteine by reacting a heat-treated plant of genus Allium with an enzyme containing protease or lactase derived from Bacillus subtilis (JP 6105871 B2), a method of obtaining S-1-propenylcysteine by coexisting squeezed juice or extract of the plant of genus Allium with an enzyme having a gamma-glutamyl transpeptidase or glutaminase activity (JP 2015-144571 A), a method of obtaining S-1-propenylcysteine in which garlic is inoculated with yeast and fermented (KP 2010-072874-5), and a method in which the plant of genus Allium is aged in an aqueous ethanol solution for one month or more to obtain S-1-propenylcysteine (WO 2016/088892 A, Molecules. 2017; 22: 570).

Here, examples of the plant of genus Allium include garlic (Allium sativum L.), onion (Allium cepa L.), elephant garlic (Allium ampeloprasum L.), Allium tuberosum, and green onion (Allium fistulosum L.). Such plants may be used solely or in combination. In addition, as the plant of genus Allium, a raw plant can be used as it is, or if necessary, be removed of its outer shell, cut or shredded, or made into powder or extract using a solvent capable of producing a medicine or food. Examples of the solvent include water and alcohol, and a solvent obtained by adding an acid or a basic substance to the solvent.

S-1-propenylcysteine or a salt thereof used in the present invention can be either an isolated and purified product, a crude product, the above plant or a processed product thereof, or a fraction in which a content of S-1-propenylcysteine or a salt thereof is increased by an extraction operation from the above plant.

The agent of the present invention may include S-1-propenylcysteine or a salt thereof in an amount of 1 ppm or more, preferably 10 ppm or more, 100 ppm or more, 500 ppm or more, or 1000 ppm or more in terms of dry matter. The upper limit of the content is not particularly limited but may be 100000 ppm or less, 50000 ppm or less, or 10000 or less.

For example, as shown below, it can be obtained by 1) extracting a plant of genus Allium in a 10 to 50% aqueous ethanol solution at 0 to 80 °C for one month or more (step 1), and 2) collecting an ethanol-eluted fraction after a solid-liquid separation of the obtained extract (step 2).

The aqueous ethanol solution used in step 1) can be a 10 to 50% aqueous ethanol solution. However, the solution is preferably prepared to have an ethanol concentration of 20 to 40%. In addition, a treatment temperature can be set within a range of 0 to 80 °C, preferably 10 to 60 °C, and more preferably 20 to 40 °C. A treatment period before extraction can be at least one month or more under the above conditions, preferably 1 to 20 months, and more preferably 1 to 10 months. In addition, this step can be performed in an airtight, sealed or tight container considering for example, hygiene and ethanol volatilization. However, it is preferable to use a tight container.

In step 2), the extract obtained in step 1) is subjected to the solid-liquid separation to collect the ethanol-eluted fraction. By appropriately concentrating the collected material, it is possible to obtain an extracted fraction containing S-1-propenylcysteine or a salt thereof. The extracted fraction can be used as it is, or be used after being appropriately dried by spray-drying or the like.

Furthermore, an isolation of S-1-propenylcysteine or a salt thereof from the extracted fraction containing S-1-propenylcysteine or a salt thereof can be realized by dialysis using a dialysis membrane with a molecular exclusion size of 3000 to 4000 as necessary, and then appropriately combining the dialysis with an adsorption/separation method using a cation exchange resin or a means of separation and purification by normal phase chromatography or reversed-phase chromatography. Here, examples of the adsorption/separation method using the cation exchange resin include adsorbing on the cation exchange resin (for example, Amberlite (manufactured by the Dow Chemical Company), DOWEX (manufactured by the Dow Chemical Company), DIAION (manufactured by Mitsubishi Chemical Corporation)) and eluting with 0.1 to 3 N ammonia water. Examples of the normal phase chromatography include a method of eluting with a chloroform/methanol/water mixture or the like using a silica gel column. Examples of the reversed-phase chromatography include a method of eluting with a 0.01 to 3% aqueous formic acid solution or the like using an octadecylsilyl column.

Preferably, a method is mentioned, wherein the ethanol-extracted fraction is dialyzed (the dialysis membrane: the molecular exclusion size 3000 to 4000), then adsorbed on a cation exchange resin, then eluted with 0.5 to 2 N aqueous ammonia, and the eluate is subjected to silica gel column chromatography (the solvent: chloroform/methanol/water mixture) to collect a fraction containing a target product, and further subjected to preparative reversed-phase column chromatography (the solvent: 0.1 to 0.5% aqueous formic acid solution) to collect the target product. The ratio of the trans isomer of S-1-propenylcysteine obtained in the above steps 1) and 2) is approximately 70 to 90% when the total of the trans isomer and the cis isomer is taken as 100%.

S-1-propenylcysteine or a salt thereof in the present invention is generally of low toxicity, because, for example, LD₅₀ value of dilute ethanolic extract of garlic (an extract content: 14.5%, an alcohol number: 1.18), which is one of the raw materials, is 50 ml/kg or more in any of oral, intraperitoneal, and subcutaneous administration routes (The Journal of Toxicological Sciences. 1984; 9: 57.), and plants of genus Allium such as garlic and onion are commonly used as food.

As shown in test examples to be described later, S-1-propenylcysteine or a salt thereof increased eNAMPT in blood. Therefore, S-1-propenylcysteine, a salt thereof, or a composition containing the same may be an eNAMPT increasing agent. An extracellular nicotinamide phosphoribosyltransferase, eNAMPT, secreted from adipose tissues and the like, circulates in body fluids such as blood, and is delivered to cells in one or more organs throughout the body to promote NAD⁺ synthesis. Actually, eNAMPT increased NAD⁺ (having a function known to improve functions of sirtuins) in animal's brain, kidney, skeletal muscle, and liver. Furthermore, eNAMPT increased the SIRT1 activity in cerebral cortexes and increased the SIRT1 expression level in hippocampi, kidneys, hypothalamuses, hearts, lungs, livers, and skeletal muscles, in senescence accelerated mice. In addition, S-1-propenylcysteine or a salt thereof suppressed cells that had expressed p16 and p21 in the kidney and p53 in the hippocampus, which were markers of senescent cells.

Comprehensively considering the kinetics and actions of the blood eNAMPT together with the phenomena of increase in blood eNAMPT, increase in NAD⁺ in each organ, activation or expression enhancement of sirtuins, and suppression of senescent cells, S-1-propenylcysteine, a salt thereof, or a composition containing the same can be administered to an animal and used for activating or enhancing expression of sirtuins, increasing NAD⁺, and suppressing senescent cells, in one or more (preferably, 2 or more, 3 or more, 4 or more, 5 or more, 6 or more, or 7 or more, independently for each application) of organs.

Examples of the organ include, but are not limited to, at least one of brain, kidney, skeletal muscle, liver, and heart. Above all, the organ is exemplified by at least one of skeletal muscle, liver, and heart, and otherwise, brain and kidney.

However, the present invention may not include an aspect in which the agent acts only on brain. The present invention may not include an aspect in which the agent acts only on kidney in activation or expression enhancement of sirtuins, and suppression of senescent cells. The present invention may not include an aspect in which the agent acts only on brain and kidney in activation or expression enhancement of sirtuins.

The sirtuin activation or expression enhancer and the senescent cell inhibitor of the present invention may be used for preventing, treating or ameliorating symptoms resulting from low activity or low expression of sirtuins, or symptoms resulting from an increase in senescent cells. As described above, functional deterioration of SIRT1 and accelerated cellular senescence are considered to be related to symptoms and diseases such as kidney injuries, cognitive/memory disorders (including short-term and long-term), liver steatosis, cardiac dysfunction, and fatty liver (Non-Patent Documents 2 to 4, 9, and 10). Examples of the kidney injuries include diseases with highly-expressed KIM-1 and NGAL, acute kidney injury, diabetic nephropathy, and nephrotic syndrome. Examples of the cognitive/memory disorders include neurological disorders in cerebral cortex or the hippocampus, learning disorders, and memory disorders (including short-term and long-term).

In the present invention, "sirtuins" mean sirtuin proteins and homologs thereof. Humans are known to have 1 to 7 sirtuins (SIRT1 to SIRT7). However, SIRT1 is preferred in the present invention.

The "sirtuin expression enhancement" means an increase in a transcript of a sirtuin gene, and/or a sirtuin protein, and includes, for example, activating transcription and/or translation of a gene, improving stability of the transcript and/or the protein, inhibiting degradation of the transcript and/or proteolysis, etc.

In the present invention, the "cellular senescence" refers to a phenomenon in which the irreversible cell cycle arrest occurs in a cell. The cellular senescence has been observed, for example, in brain cells (such as hippocampal cells and cerebral cortex cells), kidney cells, liver, and skeletal muscle. The "senescent cell suppression" refers to one or both of removal of senescent cells and prevention or delay of a cellular senescence phenomenon.

The p16, p21, and p53 genes are known as molecular markers of DNA damage that causes the cellular senescence. However, the senescent cell inhibitor of the present invention more preferably suppresses mitochondrial dysfunction that accelerates the cellular senescence and DNA damage. In addition, the senescent cells can be more effectively suppressed by enhancing the immune functions.

The NAD⁺ increasing agent of the present invention may be used for preventing, treating, or ameliorating symptoms resulting from a low NAD⁺ level. As described above, the low NAD⁺ level is considered to be related to symptoms and diseases such as nonalcoholic fatty liver diseases and kidney injuries (Non-Patent Documents 2 to 5, and 10 to 12). Examples of kidney injuries include diseases with highly-expressed KIM-1 and NGAL, acute kidney injury, diabetic nephropathy, and nephrotic syndrome.

The eNAMPT and/or NAD⁺ increasing agent, sirtuin activation/expression enhancer, and senescent cell inhibitor of the present invention may be in a form of medicine or food, or in a form of a material or a preparation added thereto.

In addition, the food includes a food product, a functional food product, a food product labeled with functionality, a food product for patients, a food product for specified health uses, and a nutritional supplement (a supplement), which are based on the concept of increases in eNAMPT and/or NAD⁺, sirtuin activation/expression enhancement, or senescent cell suppression and where an action based on the function is described and displayed as necessary.

A dosage form of the medicine is preferably a dosage form suitable for oral administration. As a specific dosage form of an oral administration preparation, for example, a solid preparation may be in a form of a tablet, a capsule, a fine particle, a pill, or a granule, and a liquid preparation may be in a form of an emulsion, a solution, a suspension, or a syrup. Such a pharmaceutical preparation can be prepared by appropriately blending, for example, an excipient, a binder, a disintegrant, a lubricant, a colorant, a flavoring agent, and a pH adjuster, into S-1-propenylcysteine or a salt thereof, as necessary, according to a conventional method. However, the dosage form of the medicine is not particularly limited, and may be a dosage form suitable for parenteral administration, for example, a dosage form suitable for intravenous (an injection and catheter), transmucosal (a liquid or ointment), or intracranial administration (a catheter).

A form of S-1-propenylcysteine food is not particularly limited, and can take various forms such as a solid food product, a semi-fluid food product, a gel-like food product, a tablet, a caplet, and a capsule, and more specifically a form of various food products such as a confection, a beverage, a seasoning, a processed fishery product, a processed meat food product, bread, and a health food product. Such food can be produced by appropriately blending a food material used usually for producing such food with S-1-propenylcysteine or a salt thereof according to the conventional method.

The medicine or food can contain other substances involved in increase in eNAMPT and/or NAD⁺, activation or expression enhancement of sirtuins, and suppression of senescent cells, for example, resveratrol or nicotinamide mononucleotide. In addition, for example, vitamins, lipids, and minerals that alleviate inflammation, such as vitamin C, vitamin E, vitamin B2, vitamin B6, niacin, hesperidin, α-lipoic acid, glutathione, coenzyme Q10, zinc, magnesium, and omega-3 fatty acid can be contained.

A preferred intake amount of the aforementioned medicine or food per day varies depending on factors, for example, subjects to be ingested, forms of ingestion, types of materials, additives to be ingested simultaneously, and ingestion intervals. However, it is preferable to ingest 0.001 to 10 mg/kg of S-1-propenylcysteine or a salt thereof per day, and it is more preferable to ingest 0.1 to 1 mg/kg per day. In addition, a daily dose can be optionally divided into 2 to 4 times for ingestion.

Examples of the subject for administration and ingestion of the medicine include living organisms with lowered activity or expression level of sirtuins, increased senescent cells, or decreased NAD⁺ and/or eNAMPT amount. However, the examples may also include healthy living organisms without these phenomena or specific diseases (e.g., diabetes, kidney injuries). The living organisms are not limited to the animals described so far, but also include nematodes and cells (may be derived from the above animals or plants). However, animals are preferable. Examples of the animals include vertebrates (preferably rodents and primates), fish, birds, insects, and reptiles, and in particular, rodents (particularly rats and mice) and primates (particularly humans and monkeys) are preferable.

As far as the present inventors have known, there is no proven fact that a processed product of the plant of genus Allium or the sulfur-containing component contained in the plant of genus Allium is administered to an animal to increase eNAMPT, activate sirtuins or enhance sirtuin expression, and increase NAD⁺ in an animal body, and no proven fact that the processed product or the sulfur-containing component suppress senescence of non-cancer cells in the animal body. These facts were unexpectedly discovered as a result of intensive studies by the present inventors.

Examples of the living organisms include humans suffering from e.g., kidney injuries, cognitive/memory disorders (including short and long terms), liver steatosis, cardiac dysfunction, fatty liver, and nonalcoholic fatty liver disease, or animals (for example, humans) hoping to prevent the diseases. However, healthy living organisms are also preferable.

### <Other aspects>

In another aspect, the present invention may also relate to S-1-propenylcysteine or a salt thereof, or a composition containing S-1-propenylcysteine or a salt thereof, which are used for the various applications described above. In another aspect, the present invention may also relate to a method for administering S-1-propenylcysteine or a salt thereof, or a composition containing S-1-propenylcysteine or a salt thereof, for use in the various applications described above. In another aspect, the present invention may also relate to a use of S-1-propenylcysteine or a salt thereof, or a composition containing S-1-propenylcysteine or a salt thereof, for the various applications described above. In another aspect, the present invention may also relate to a use of S-1-propenylcysteine or a salt thereof, or a composition containing S-1-propenylcysteine or a salt thereof, in producing the agent for use in the various applications described above. The details of these aspects are the same as those of the agent described above.

### EXAMPLES

### Preparation Example 1 Ethanol-Extracted Fraction of Garlic

About 1 kg of peeled garlic bulbs and about 1000 mL of 30% ethanol were put in a container and sealed. The container was left at room temperature for 1 to 10 months, and stirred as appropriate. Solid and liquid were separated from the mixture, and the liquid was dried by spray-drying to obtain a yellow-brown powder.

### Preparation Example 2 Separation of S-1-Propenylcysteine from the Ethanol-Extracted Fraction of Garlic

(1) The ethanol-extracted fraction of garlic obtained in Preparation Example 1 was put into a dialysis tube with a pore size of 3500, and dialyzed against purified water. The dialysate was passed through a cation exchange resin Dowex 50Wx8 (H+), and the resin was thoroughly washed with purified water. Amino acids adsorbed to the resin were eluted with 2 N ammonia and concentrated under reduced pressure. A concentrate was applied to a silica gel column, and subjected to column chromatography using a chloroform/methanol/water mixture as a solvent. The fraction containing the target product (S-1-propenylcysteine) were collected and concentrated. The concentrate was dissolved in water, and chromatography was performed using a preparative reversed-phase column (the octadecylsilyl column) with 0.1% formic acid as a solvent to collect the target product, and the solvent was removed by freeze drying. The obtained lyophilizate was confirmed to be a mixture of trans-S-1-propenylcysteine and cis-S-1-propenylcysteine (trans isomer: cis isomer=8: 2) by comparing the structure with spectra obtained from the following standard substances using NMR (solvent: heavy water) and a mass spectrometer.

### Trans-S-1-propenylcysteine

¹H-NMR (500 MHZ, in D₂O-NaOD, δ): 1.76 (d, 3H, J=7.0 Hz), 2.98 (dd, 1H, J=7.5, 14.5 Hz), 3.14 (dd, 1H, J=4.5, 14.5 Hz) 3.69 (dd, 1H, J=4.5, 7.5 Hz), 5.10-5.14 (m, 1H), 6.02 (d, 1H, J=15.5 Hz);
¹³C-NMR (125 MHz, in D₂O-NaOD, δ): 17.61, 33.53, 53.70, 119.92, 132.12, 172.73,
HRMS: observed [M+H]⁺=162.0583, calculated [M+H]⁺=162.0581.

### Cis-S-1-propenylcysteine

¹H-NMR (500 MHz, in D₂O, δ): 1.74 (d, 3H, J=7.0 Hz), 3.21 (dd, 1H, J=7.5, 15.0 Hz), 3.31 (dd, 1H, J=4.5, 15.0 Hz), 3.95 (dd, 1H, J=4.5, 7.5 Hz), 5.82-5.86 (m, 1H), 6.01 (d, 1H, J=9.5 Hz); ¹³C-NMR (125 MHz, in D₂O-NaOD, δ): 13.89, 33.88, 54.16, 122.58, 127.78, 172.63.

HRMS: observed [M+H]⁺=162.0580, calculated [M+H]⁺=162.0581.

### (2) Measurement of S-1-Propenylcysteine in the Ethanol-Extracted Fraction of Garlic

500 mg to 1 g of the ethanol-extracted fraction of garlic obtained in Preparation Example 1 (1) was put in a container, and 20 mM hydrochloric acid solution of S-n-3 butenyl cysteine was added thereto as an internal standard, and the volume was adjusted to 20 mL with 20 mM hydrochloric acid. After the mixture was well stirred, a part thereof was taken and centrifuged at 1750 G for about 10 minutes. A part of the supernatant obtained was taken and subjected to centrifugal filtration using a centrifugal filtration unit (Amicon Ultra, cutoff: 3000) (15000 rpm, 10 min). 20 µL of the obtained filtered product was taken and derivatized using an AccQ-Tag Derivatization Kit (Waters). Separately, a standard compound was dissolved in 20 mM hydrochloric acid, and the same procedure as for a sample was performed to prepare a standard solution for calibration curve. A sample solution and the standard solution were chromatographed on an Acquity UPLC system (Waters) to determine the content. As a result, S-1-propenylcysteine was of 3.7±0.3 mg/g dry matter.

### Test Examples

### Sirtuin activation action

### (1) Sample preparation

Preparation of a test liquid for evaluating biological activity was performed as follows. When evaluating the biological activity, all test liquids were prepared at the time of use.
(a) About 5 mg of S-1-propenylcysteine (cis/trans mixture) produced in Preparation Example 2 was weighed precisely, and dissolved in 10 mL of purified water to prepare an administration liquid.
(b) About 6 mg of S-1-propenylcysteine (cis/trans mixture) produced in Preparation Example 2 was weighed precisely and dissolved in 1 mL of culture liquid. The liquid was used as a stock solution, diluted appropriately, and subjected to an in vitro test.

### (2) Animals for Evaluation Tests

Animals for evaluating the biological activity were bred as follows. Senescence-accelerated mice SAMP8 (male) for testing were purchased from Japan SLC, Inc. After purchase, the animals were acclimatized for one week and used as animals for the evaluation tests.

### (3) Preparation of Human Neuroblasts for Evaluation Tests

Human neuroblast line SH-SY5Y cells were cultured in Dulbecco's Modified Eagle's Medium added with 10% fetal bovine serum, antibiotic penicillin, and streptomycin solution, and were used as cells for the evaluation tests.

### (4) Sirtuin Activation Test

A SIRT1_GLO kit manufactured by Promega Corporation was purchased to measure the sirtuins activity. A lysate of the cerebral cortex was placed in a 96-well plate, various reaction reagents were added, and then luminous intensity of luciferase was measured by a plate reader.

### (a) SIRT1 Activation Action (In Vivo):

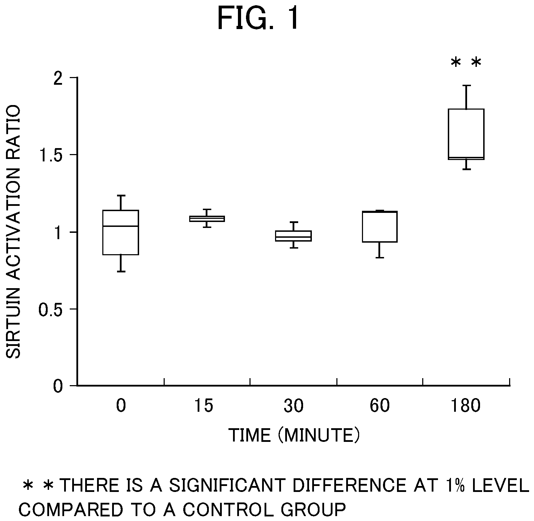

A single dose of the sample prepared in the above (1) (a) was orally administrated to animals for the evaluation tests in the above (2), and then cerebral cortices of such animals were extracted 15, 30, 60, and 180 minutes later. The cerebral cortices were crushed using an automatic tissue crusher and lysed by adding a cell dissolution liquid. Centrifugation was performed and the sirtuins activity in the supernatant thereof was measured. Results are shown in FIG. 1. The sirtuin activity increased 180 minutes after administrating S-1-propenylcysteine. (Fig. 1).

### (b) SIRT1 Protein Increasing Action (In Vivo):

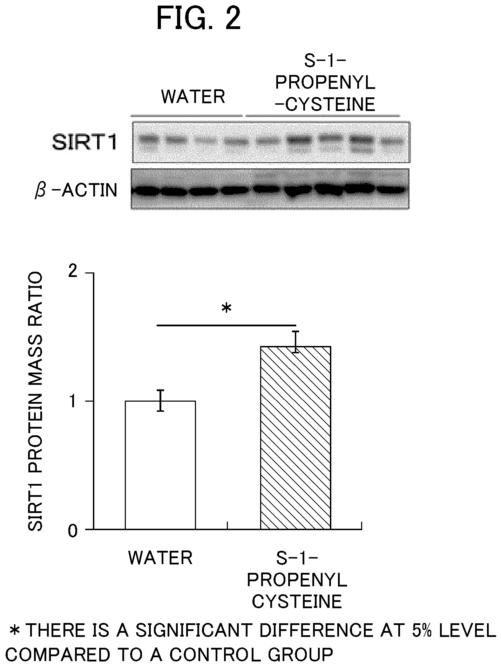

After repeated oral administration of the sample prepared in the above (1) (a) to animals for the evaluation tests in the above (2) for two weeks, hippocampi of such animals were excised. After the hippocampi were crushed using an automatic tissue crusher, the cells were lysed with a RIPA cell lysis buffer manufactured by Millipore Corporation which was diluted 10-fold with purified water added with combined protease/phosphatase inhibitors manufactured by Thermo Fisher Scientific K.K., and then centrifuged (10,000 rpm, 10 min, 4° C.), and the supernatant was used as a cell extract. The cell extract was used for analysis by Western Blotting according to a conventional method. An anti-SIRT1 antibody (manufactured by Biolegend) and an anti-β-actin antibody (manufactured by Fujifilm Wako Pure Chemical Corporation) were used as antibodies. Results are shown in FIG. 2. S-1-propenylcysteine increased the SIRT1 protein mass.

### (C) NAD⁺ Amount Increasing Action (In Vivo):

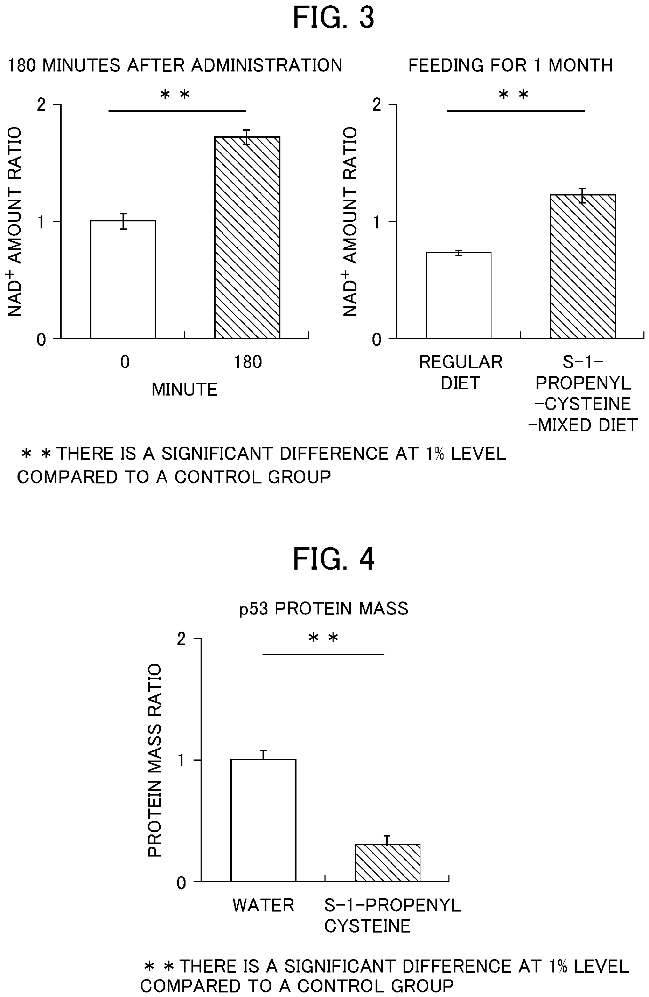

A single dose of the sample prepared in (1) (a) was orally administered to animals for the evaluation tests in the above (2), and then cerebral cortices of such animals were excised 180 minutes later. In addition, the sample prepared in the above (1) (a) was mixed with food and fed to animals for the evaluation tests in the above (2) for one month, and then cerebral cortices of such animals were excised. The NAD⁺ amount in the cerebral cortices was measured using NAD⁺/NADH Assay Kit manufactured by Dojindo Laboratories. Results are shown in FIG. 3. S-1-propenylcysteine increased NAD⁺ amount.

### (5) Suppression Action on Expression of Cellular Senescence Marker p53 Protein in the Hippocampus (In Vivo):

After repeated oral administration of the sample prepared in the above (1) (a) to animals for the evaluation tests in the above (2) for six weeks, hippocampi of such animals were excised. After the hippocampi were crushed using an automatic tissue crusher, the cells were lysed with a RIPA cell lysis buffer manufactured by Millipore Corporation which was diluted 10-fold with purified water added with a protease inhibitor and a phosphatase inhibitor manufactured by Roche, and then centrifuged (10,000 rpm, 10 min, 4° C.), and the supernatant was used as a cell extract. The cell extract was used for analysis by Western Blotting according to a conventional method. An anti-p53 antibody (manufactured by Proteintech Group, Inc.) and an anti-β-actin antibody (manufactured by Medical and Biological Laboratories Co., Ltd.) were used as antibodies. Results are shown in FIG. 4. S-1-propenylcysteine reduced a p53 protein mass.

### (6) Enhancement action of SIRT1 gene expression in kidney (in vivo):

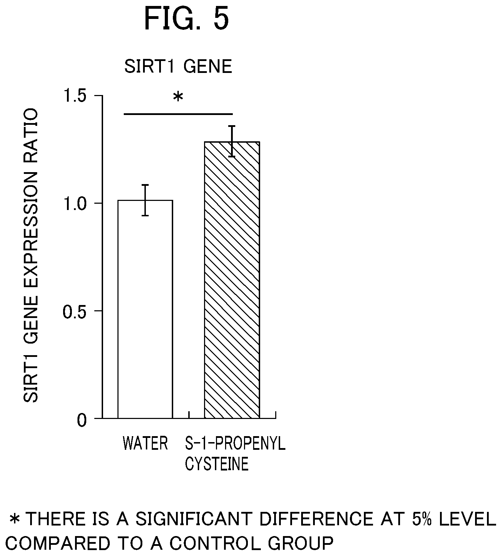

After repeated oral administration of the sample prepared in the above (1) (a) to the animals for the evaluation tests in the above (2) for two weeks, kidneys of such animals were excised. The kidneys were crushed using an automatic tissue crusher, and then RNA was extracted using a TRIzol solution manufactured by Thermo Fisher Scientific K.K.
cDNA was synthesized using PRIMEScript RT reagent Kit with gEraser manufactured by Takara. The synthesized cDNA was subjected to real-time PCR using KAPA SYBR Fast qPCR kit manufactured by Nippon Genetics Co., Ltd. Results are shown in FIG. 5. S-1-propenylcysteine increased the SIRT1 expression in the mouse kidney.

### (7) Suppression Action on Expression of Senescent Cell Markers p16 and p21 Genes in the Kidney (In Vivo):

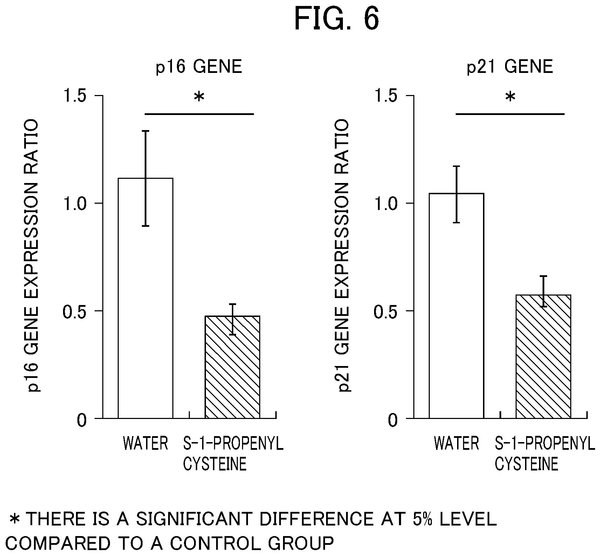

After repeated oral administration of the sample prepared in the above (1) (a) to the animals for the evaluation tests in the above (2) for two weeks, kidneys of such animals were excised. The kidneys were crushed using an automatic tissue crusher, and then RNA was extracted using a TRIzol solution manufactured by Thermo Fisher Scientific K.K. cDNA was synthesized using PRIMEScript RT reagent Kit with gEraser manufactured by Takara. The synthesized cDNA was subjected to real-time PCR using KAPA SYBR Fast qPCR kit manufactured by Nippon Genetics Co., Ltd. Results are shown in FIG. 6. S-1-propenylcysteine reduced cells expressing p16 and p21 genes in the kidneys of senescence-accelerated mice.

### (8) Inhibitory Action on Kidney Injuries (In Vivo)

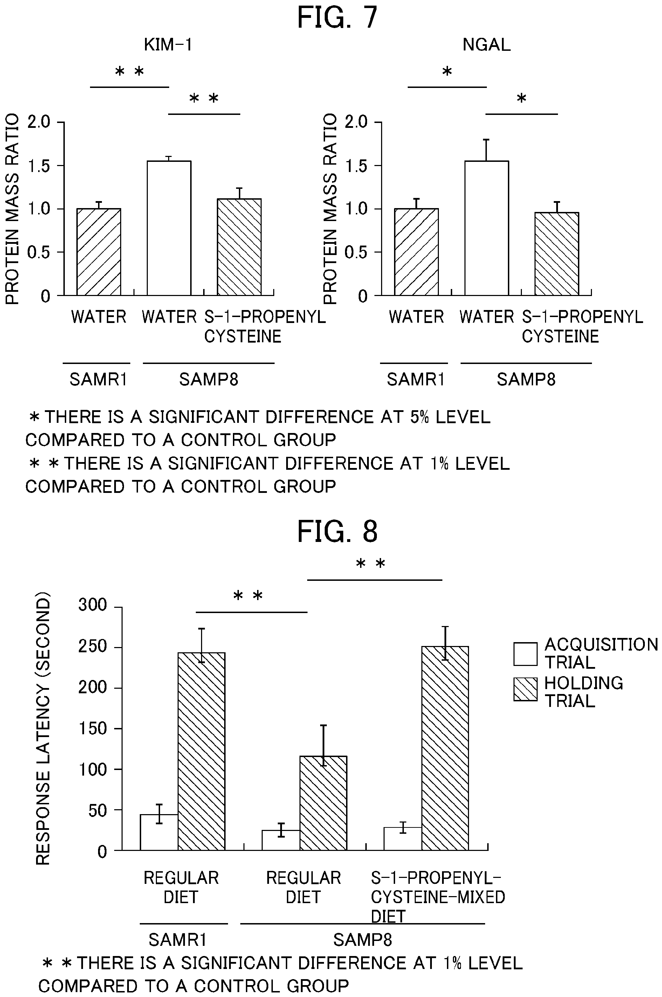

After repeated oral administration of the sample prepared in the above (1) (a) to the animals for the evaluation tests in the above (2) for two weeks, kidneys of such animals were excised. After the kidneys were crushed using an automatic tissue crusher, the cells were lysed with an RIPA cell lysis buffer manufactured by Millipore Corporation which was diluted 10-fold with purified water added with a protease inhibitor and a phosphatase inhibitor manufactured by Roche and then centrifuged (10,000 rpm, 10 min, 4° C.), and the supernatant was used as a cell extract. The cell extract was used for analysis by Western Blotting according to a conventional method. A kidney injury molecule (KIM-1) antibody (manufactured by R&D System) and a lipocalin-2 (NGAL) antibody (manufactured by Proteintech Group, Inc.), KIM-1 and NGAL being markers of kidney injuries, and an anti-GAPDH antibody (manufactured by Fujifilm Wako Pure Chemical Corporation) were used as antibodies. Results are shown in FIG. 7. S-1-propenylcysteine reduced KIM-1 and NGAL.

### (9) Cognitive/Memory Retention Action (In Vivo):

The sample prepared in the above (1) (a) was mixed with food and fed to animals for evaluation test in the above (2) for four months, and then a passive avoidance test was performed. The passive avoidance test is a test utilizing a habit that a mouse prefers a dark place, and on Day 1, a mouse already in a bright room was moved to a dark room while receiving an electrical shock (50 V/1 second), and the time it took for the mouse to enter the dark room was taken as response latency of an acquisition trial. After two months, the mouse was put in the bright room again and the time it took to move to the dark room (the response latency of a holding trial) was used as an index of memory. Results are shown in FIG. 8. S-1-propenylcysteine ameliorated aging-related decline in cognitive and memory functions.

### (10) NAD⁺ amount increasing action in kidney, skeletal muscle, and liver (in vivo):

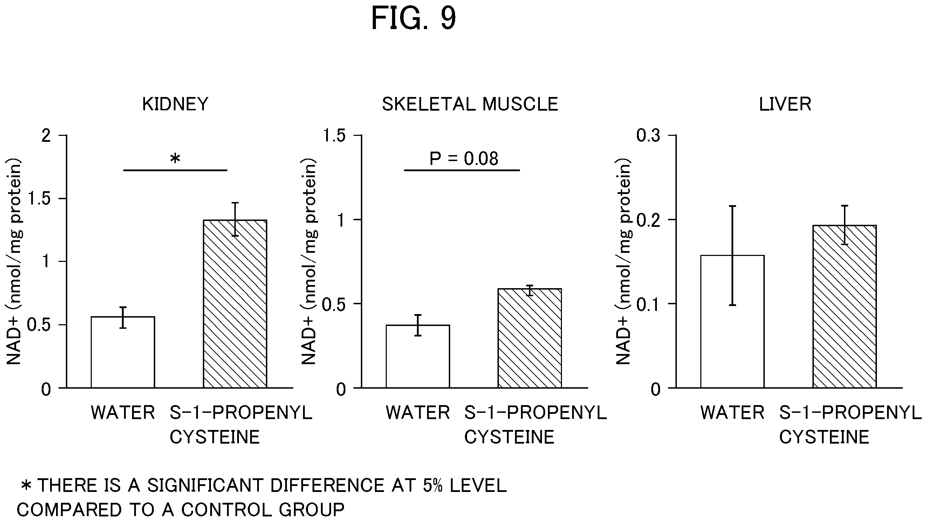

A single dose of the sample prepared in the above (1) (a) was orally administrated to animals for the evaluation tests in the above (2), and then cerebral cortexes, livers, kidneys, and skeletal muscles of such animals were extracted 60 minutes later. The NAD⁺ amounts in the cerebral cortexes, livers, kidneys, and skeletal muscles were measured using NAD⁺/NADH Assay Kit manufactured by Dojindo Laboratories. Results are shown in Fig. 9. S-1-propenylcysteine increased the NAD⁺ amount.

### (11) SIRT1 protein increasing action in cerebral cortex, hippocampus, heart, lung, liver, kidney, and skeletal muscle (in vivo):

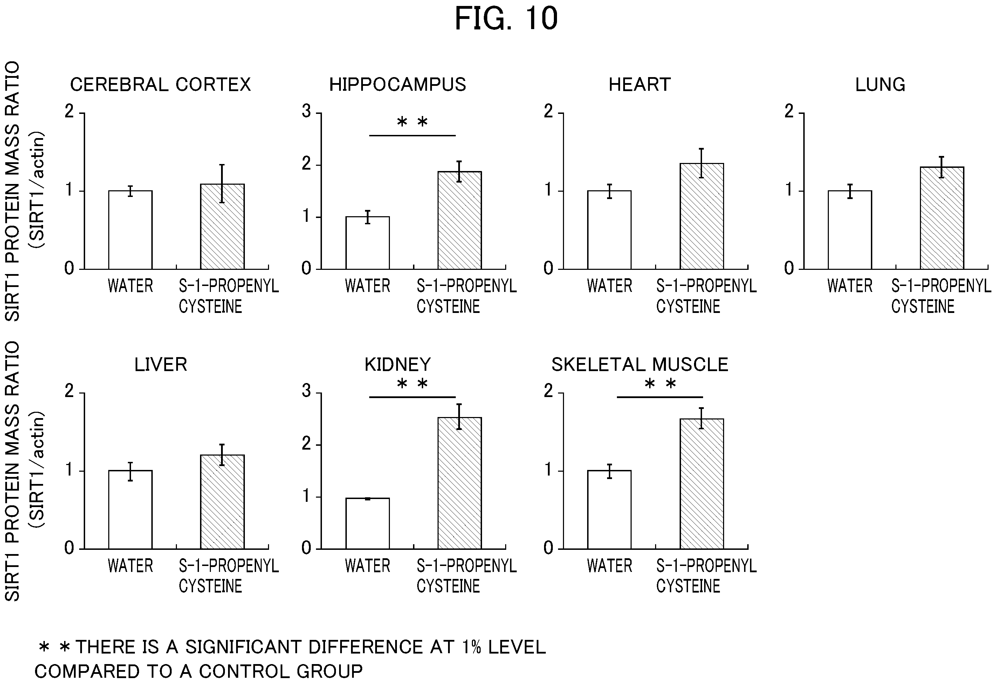

About 10 mg of S-1-propenylcysteine (cis/trans mixture) produced in Preparation Example 2 was weighed precisely, and dissolved in 10 mL of purified water to prepare an administration liquid. After repeated oral administration of the prepared sample to animals for the evaluation tests in the above (2) for two weeks, cerebral cortexes, hippocampi, hearts, lungs, livers, kidneys, and skeletal muscles of such animals were excised. After the cerebral cortexes, hippocampi, hearts, lungs, livers, kidneys, and skeletal muscles were crushed using an automatic tissue crusher, the cells were lysed with a RIPA cell lysis buffer manufactured by Millipore Corporation which was diluted 10-fold with purified water added with combined protease/phosphatase inhibitors manufactured by Thermo Fisher Scientific K.K., and then centrifuged (10,000 rpm, 10 min, 4°C), and the supernatant was used as a cell extract. The cell extract was used for analysis by Western Blotting according to a conventional method. An anti-SIRT1 antibody (manufactured by Biolegend) and an anti-β-actin antibody (manufactured by Fujifilm Wako Pure Chemical Corporation) were used as antibodies. Results are shown in Fig. 10. S-1-propenylcysteine increased the SIRT1 protein mass.

### (12) SIRT1 protein increasing action in hypothalamus (in vivo):

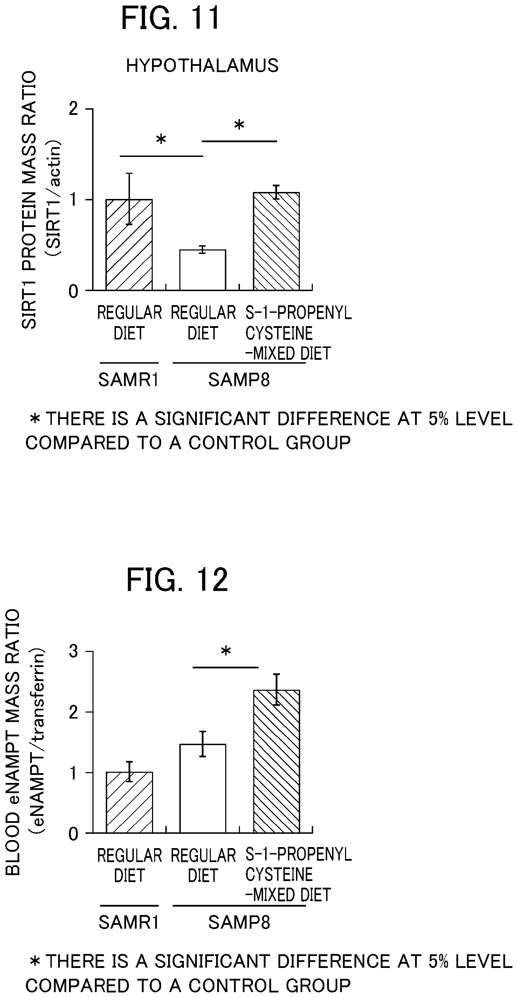

S-1-propenylcysteine (cis/trans mixture) produced in Preparation Example 2 was dissolved at a concentration of 0.001% in purified water to prepare an administration liquid. Animals for evaluating the biological activity were bred as follows. Senescence-accelerated mice SAMR1 (male) and SAMP8 (male) for testing were purchased from Japan SLC, Inc. After purchase, the animals were acclimatized for one week and used as animals for the evaluation tests. These animals for the evaluation tests were fed with regular diet and SAMP8 were fed with the diet mixed with the sample prepared above for 10 months, and then hypothalamuses of such animals were excised. After the hypothalamuses were crushed using an automatic tissue crusher, the cells were lysed with a RIPA cell lysis buffer manufactured by Millipore Corporation which was diluted 10-fold with purified water added with combined protease/phosphatase inhibitors manufactured by Thermo Fisher Scientific K.K., and then centrifuged (10,000 rpm, 10 min, 4°C), and the supernatant was used as a cell extract. The cell extract was used for analysis by Western Blotting according to a conventional method. An anti-SIRT1 antibody (manufactured by Biolegend) and an anti-β-actin antibody (manufactured by Fujifilm Wako Pure Chemical Corporation) were used as antibodies. Results are shown in Fig. 11. S-1-propenylcysteine increased the SIRT1 protein mass.

### (13) eNAMPT increasing action in blood (in vivo):

In the animals from which hypothalamuses had been excised in (12), the blood eNAMPT amounts were measured according to the method described in Yoshida. M. et.al., Cell Metab., 2019; 30(2): 329-342. e5. Results are shown in Fig. 12. S-1-propenylcysteine increased the blood eNAMPT amount.

### (14) SIRT1 protein mass increasing action in liver and heart (in vivo):

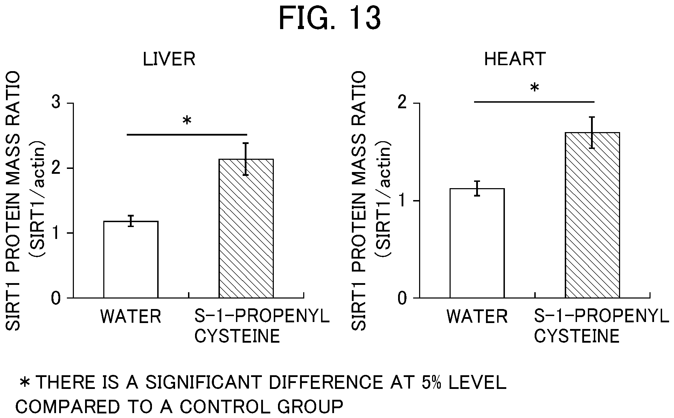

After repeated oral administration of the sample prepared in the above (1) (a) to evaluation test animals prepared in the same manner as in (12) for two weeks, livers and hearts of such animals were excised. After the livers and hearts were crushed using an automatic tissue crusher, the cells were lysed with a RIPA cell lysis buffer manufactured by Millipore Corporation which was diluted 10-fold with purified water added with combined protease/phosphatase inhibitors manufactured by Thermo Fisher Scientific K.K., and then centrifuged (10,000 rpm, 10 min, 4°C), and the supernatant was used as a cell extract. The cell extract was used for analysis by Western Blotting according to a conventional method. An anti-SIRT1 antibody (manufactured by Biolegend) and an anti-β-actin antibody (manufactured by Fujifilm Wako Pure Chemical Corporation) were used as antibodies. Results are shown in Fig. 13. S-1-propenylcysteine increased the SIRT1 protein mass.

## Claims

1. An eNAMPT increasing agent comprising S-1-propenylcysteine or a salt thereof as an active ingredient.

2. An eNAMPT increasing agent comprising S-1-propenylcysteine or a salt thereof that is administered to an animal.

3. The agent according to claim 2, which is used for increasing eNAMPT in blood.

4. An agent comprising S-1-propenylcysteine or a salt thereof that is administered to an animal and is used for activating or enhancing expression of sirtuins, or suppressing senescent cells in one or more organs.

5. An agent comprising S-1-propenylcysteine or a salt thereof that is administered to an animal and is used for increasing NAD⁺ in one or more organs.

6. The agent according to claim 4 or 5, wherein the organ comprises at least one selected from the group consisting of brain, kidney, skeletal muscle, liver, and heart.

7. The agent according to claim 6, wherein the organ comprises at least one selected from the group consisting of skeletal muscle, liver, and heart.

8. The agent according to claim 7, wherein the organ further comprises at least one selected from the group consisting of brain and kidney.

9. The agent according to claim 4 or 5 for acting on two or more organs.

10. The agent according to claim 9 for acting on three or more organs.

11. The agent according to any one of claims 1, 4, and 5 being in a form of a food additive or food.
